# EUROPÄISCHE PATENTANMELDUNG

(11) **EP 3 106 178 A1**
(43) Veröffentlichungstag der Anmeldung: **21.12.2016**
(21) Anmeldenummer: 16173803.4
(22) Anmeldetag: 09.06.2016
(51) Int. Cl.: A61L 2/20, B01D 29/11, F01L 9/04, A61L 2/00, A61L 2/02, A61L 9/12

(54) **KERZENFILTER-GEHÄUSE**

(30) Priorität: 09.06.2015 CH 8212015
(71) Anmelder: Ervo Immobilien GmbH, 6714 Nüziders (AT)
(72) Erfinder: Willi, Erhard, 6782 Silbertal (AT)
(74) Vertreter: Riederer Hasler & Partner Patentanwälte AG

(57) **Zusammenfassung**

Die Erfindung betrifft ein Kerzenfilter-Gehäuses (A, B, C, D) zur Herstellung eines sterilen Mediums mit einer austauschbaren Filterkerze (13), einem Filterfuss (25) an welchem die Filterkerze (13) austauschbar gehalten ist und an welchem eine Zulauf (15) und ein Ablauf (17) für das Medium vorhanden ist, einer Gehäuseglocke (28), in welcher die Filterkerze (13) dichtend aufgenommen ist und welche an dem Filterfuss (25) abnehmbar befestigt ist und einer Bypass-Leitung (29), welche den Zulauf (15) und den Ablauf (17) miteinander verbindet. Die Bypass-Leitung (29) ist in dem Filterfuss (25) ausgebildet ist.

## Beschreibung

### Gebiet der Erfindung

Die Erfindung betrifft ein Kerzenfilter-Gehäuse zur Herstellung eines sterilen Mediums gemäss Oberbegriff des Anspruchs 1 und eine Filtergruppe gemäss den Ansprüchen 11 und 12.

### Stand der Technik

Kerzenfilter-Gehäuse werden bei beim Filtrieren von Flüssigkeiten in einer Vielzahl von Anwendungsgebieten eingesetzt. Dazu zählen auch die pharmazeutische und chemische Industrie. In der pharmazeutischen Industrie sind oftmals weniger ein hoher Durchsatz als ein hoher Reinheitsgrad gefordert. In der pharmazeutischen Sterilfiltration werden Kerzenfilter-Gehäuse mit Filterkerzen mit einer Porengrösse von ca. 0,22 µm verbreitet eingesetzt. Ein Kerzenfilter-Gehäuse, welches zur Sterilisierung eines flüssigen Produktes zum Einsatz kommt, ist vor jedem Prozesszyklus selbst mit Heissdampf von wenigstens 121° C zu sterilisieren.

Filterkerzen mit einer für sterile Pharmaprodukte notwendigen Filtrationsleistung ertragen lediglich einen Differenzdruck von weniger als 0,2 bar zwischen der Fluideingangs- und der Fluidausgangsseite. Um diesen Differenzdruck bei CIP (cleaning in place), DIP (drying in place) und SIP (sterilization in place) Reinigungsverfahren nicht zu überschreiten, welcher zu einer Zerstörung der Filterkerze führen würde, behilft man sich mit Bypass-Leitungen. Durch Öffnen und Schliessen von Ventilen in den Bypass-Leitungen lässt sich z.B. der Heissdampf unter dem kritischen Differenzdruck von 0,2 bar halten.

Nachteilig an dieser Regelung ist jedoch, dass die Bypass-Leitungen zwangsläufig zusätzliche unerwünschte Toträume und Rohrstrecken in den Aufbau des Kerzenfilters einbringen. Insbesondere ist die Abfüllvorrichtung, welche das sterile Medium in die Gebrauchsgebinde abfüllt, von dem Kerzenfilter-Gehäuse wenigstens um die Länge eines T-Stückes beabstandet. Das T-Stück ist vorzusehen, damit die Bypass-Leitung in die Produktleitung zurückgeführt wird.

Ist eine Dampfsterilisation des Gesamtsystems durchzuführen, sind die maximal mögliche Druckdifferenz des eingesetzten Filters zu beachten. Um den vorgegebenen Differenzdruck nicht zu überschreiten, muss eine zusätzliche Einleitung des Dampfs nach der Sterilfiltration vorgesehen sein. Nachteilig sind nach dem Filter eingebrachte Ventile und Toträume, deren Tiefe systembedingt grösser 3 x dem angrenzenden Rohrdurchmesser D sein müssen. Ferner können Ventile, welche nach der Sterilfiltration eingebaut sind, nicht auf Fehlfunktion überwacht werden. Eine Leckage eines Dichtsitzes oder Druckspitzen, welche zur Öffnung der nachgeschalteten Ventile führen, sind daher ein potentielles Risiko.

### Aufgabe der Erfindung

Aus den Nachteilen des beschriebenen Stands der Technik resultiert die die vorliegende Erfindung initiierende Aufgabe ein gattungsgemässes Kerzenfilter-Gehäuses weiterzuentwickeln, welches möglichst kompakt ist und dementsprechend wenige Leitungsstrecken und Toträume aufweist. Noch ein Ziel der Erfindung ist es, die Dichtheit der Bypass-Leitung gegenüber der Produktleitung möglichst einfach überwachen zu können.

### Beschreibung

Die Lösung der gestellten Aufgabe gelingt bei einem Kerzenfilter-Gehäuse dadurch, dass die Bypass-Leitung in dem Filterfuss ausgebildet ist. Die Bypass-Leitung besitzt durch ihre Integration in den Filterfuss sehr kurze Wege, welche unter die Anforderungen fallen, dass die Totlängen (Toträume) kürzer sind als das Dreifache der an die Totlänge angrenzenden Rohr- bzw. Bohrungsdurchmesser. Der Kerzenfilter-Gehäuse ist daher sehr einfach inline zu reinigen und bietet einen hohen Hygienestandard. Reste von Reinigungsmittel oder abgesetzten Produkt sind dadurch vollständig ohne zusätzlichen Aufwand entfernbar.

Die Erfindung zeichnet sich bevorzugt dadurch aus, dass die Bypass-Leitung durch eine Mehrzahl von an dem Filterfuss vorgesehenen Bohrungen und/oder Fräsungen realisiert ist. Da der Filterfuss ohnedies bevorzugt aus Metall gefertigt ist und seine Form durch Fräs- und Bohrvorgänge erhält, lässt sich die Bypass-Leitung ohne grossen zusätzlichen Aufwand in den Filterfuss integrieren.

In einer besonders bevorzugten Ausführungsform der Erfindung ist in die Bypass-Leitung wenigstens ein Öffnungs- bzw. Schliessventil integriert, welches derart ausgelegt ist, dass es beim maximal auftretenden Mediendruck im geschlossenen Zustand den Zulauf und den Ablauf gegenüber der Bypass-Leitung verschliesst. Bei jedem erreichbaren Mediendruck dichtet das Ventil den Mediumkreislauf während der Sterilfiltration gegenüber der der Umgebung offenen Bypass-Leitung, um das Medium steril zu halten. Da das Kerzenfilter-Gehäuse gemäss der Erfindung zur Herstellung eines sterilen Mediums dient, ist es von grösster Bedeutung, dass der Mediumkreislauf dicht ist, damit keine Verunreinigungen wie Keime oder Bakterien über die Bypass-Leitung in das sterile Medium während der Produktion gelangen. Das Ventil darf sich demnach nur dann von aussen öffnen lassen, wenn dies für Reinigungszwecke beabsichtigt ist. Überdruckventile oder Ventile, welche sich durch den Mediendruck und andere Betriebsparameter als dem Mediendruck öffnen lassen, sind daher für das vorliegende Kerzenfilter-Gehäuse völlig ungeeignet. Das automatische Öffnen des Ventils, was aus Sicherheitsgründen erwünscht sein kann, würde bei der vorliegenden Erfindung zur Zerstörung der gesamten Charge des sterilen Mediums führen. Die in dem Kerzenfilterfuss integrierte Bypass-Leitung dient daher nicht der Umgehung des Kerzenfilters während der Produktion des sterilen Mediums sondern der Umgehung während der Reinigung, um den auf den Kerzenfilter wirkenden Druck zu reduzieren und eine Zerstörung des Kerzenfilters während der Reinigung zu verhindern.

Als zweckdienlich hat es sich erwiesen, wenn an dem Filterfuss wenigstens ein Ventilsitz ausgeformt ist, welcher mit einem ersten Membranventil derart zusammen wirkt, dass die Bypass-Leitung geschlossen und geöffnet werden kann. Der Ventilsitz ist ebenfalls in den Filterfuss gefräst. Dies lässt sich auf CNC- Fräsmaschinen sehr rasch und mit grosser Präzision realisieren. Dementsprechend exakt kann der Ventilsitz mit dem Membranventil zusammenwirken. Ferner können an dem Filterfuss auch alle Haltebohrungen für das Membranventil vorgenommen werden. Membranventile lassen sich fein dosieren und sind für eine CIP-Reinigung gut geeignet, da sie selbstentleerend sind.

Zweckmässigerweise ist der Filterfuss aus dem Vollen gearbeitet. Dadurch können Undichtigkeiten in dem Filterfuss ausgeschlossen werden und Passungen und Abmessungen sind mit der notwendigen Präzision herstellbar.

Als vorteilhaft erweist es sich, wenn der Filterfuss einstückig ist. Der Filterfuss kann daher in einem Arbeitsgang auf einer CNC-Fräsmaschine hergestellt werden. Abdichtungen zwischen mehreren Bauteilen können entfallen. Ausserdem sind keine Schweissnähte notwendig.

Die Erfindung zeichnet sich auch bevorzugt dadurch aus, dass dem Filterfuss eine Leckage-Leitung vorgesehen ist, welche mit der Bypass-Leitung in Verbindung steht. Bevorzugt ist die Leckage-Leitung am tiefsten Punkt der Bypass-Leitung angeordnet. Die Leckage-Leitung zeigt zu jedem Zeitpunkt während des Filtervorgangs zur Herstellung eines sterilen Mediums die Dichtigkeit der Bypass-Leitung an, indem in die Leckage-Leitung gelangtes Produkt durch Ausfliessen unmittelbar erkennbar ist. So sind Undichtigkeiten, Fehlschaltungen oder Überdrücke in Echtzeit detektierbar. Während der Reinigung und Sterilisation des Kerzenfilter-Gehäuses und des Kerzenfilters sind die Leckage-Leitung zu schliessen und die ersten Membranventile zu öffnen. Bei der Reinigung ist eine Anzeige der Undichtigkeit der ersten Membranventile bzw. der Bypass-Leitung nicht notwendig. Die Leckage-Leitung dient auch als Entleerungsleitung, da sie am tiefsten Punkt des Kerzenfilter-Gehäuses angeordnet ist. Dadurch ist das Kerzenfilter-Gehäuse selbstentleerend.

In einer weiteren besonders bevorzugten Ausführungsform ist die Leckage-Leitung in dem Filterfuss ausgebildet. Dieses technische Merkmal führt zu einem kompakten Leitungssystem mit kurzen Wegen, welches vollständig in dem Filterfuss integriert ist.

Zweckmässigerweise ist die Leckage-Leitung durch eine Mehrzahl von an dem Filterfuss vorgesehenen Bohrungen und/oder Fräsungen realisiert. Dadurch lässt sich der Filterfuss, die Bypass-Leitung und die Leckage-Leitung auf einer einzigen CNC-Maschine herstellen. Auch lassen sich alle notwendigen Halterungen, beispielsweise die Innengewinde zur Halterung der Membranventile, in einem Arbeitsschritt herstellen.

In einer weiteren bevorzugten Ausführungsform weist die Bypass-Leitung einen tiefsten Punkt auf, an welchem die Leckage-Leitung mit der Bypass-Leitung verbunden ist. Dies birgt den Vorteil in sich, dass die Bypass-Leitung selbstentleerend ist und in der Bypass-Leitung vorhanden Flüssigkeit diese über die Leckage-Leitung verlassen muss, wenn das Membranventil der Leckage-Leitung geöffnet ist.

Wegen den bereits erwähnten Vorteilen von Membranventilen ist es bevorzugt, wenn die Leckage-Leitung mit einem zweiten Membranventil geöffnet und geschlossen werden kann.

In einer weiteren besonders bevorzugten Ausführungsform der Erfindung weist der Filterfuss Toträume auf, deren Tiefe kleiner als der dreifache Durchmesser der angrenzenden Bohrungsdurchmesser ist. Dadurch ist das Kerzenfilter-Gehäuse besonders einfach zu reinigen und lässt sich vollständig von Restmedium und Reinigungsflüssigkeiten reinigen.

Da die Leckage-Leitung am tiefsten Punkt des Filterfusses vorgesehen ist, ist es bevorzugt, die Leckage-Leitung als Kondensatableitung zu nutzen. Das Kondensat des sterilen Reinigungsdampfes kann aus dem Filterfuss daher nahezu vollständig abfliessen.

Zweckmässigerweise ist das Kerzenfilter-Gehäuse selbstentleerend. Diese wird dadurch erreicht, dass die Bypass-Leitung ein Gefälle aufweist und die Leckage-Leitung am tiefsten Punkt der Bypass-Leitung angeordnet ist. Reinigungsmittel und Sterildampfkondensat können daher nach der Sterilreinigung des Kerzenfilter-Gehäuses ohne weitere Hilfsmittel vollständig aus dem Kerzenfilter-Gehäuse abfliessen.

Ein weiterer Aspekt der Erfindung betrifft eine Filtergruppe mit wenigstens einem ersten und zweiten in Serie geschalteten Kerzenfilter-Gehäuse gemäss einem der vorangehenden Ansprüche, wobei der erste Filter ein Vorfilter ist und der zweite Filter ein Sterilfilter ist. Das erfinderische Kerzenfilter-Gehäuse kann daher für alle in einer Filtergruppe eingesetzten Filter zur Anwendung kommen, da Filterkerzen mit unterschiedlichen Porengrössen mit dem Filterfuss verbindbar sind. Dadurch wird die Anzahl der Toträume in einer Filter-serie auf ein Mindestmass reduziert.

Noch ein Aspekt der Erfindung betrifft wenigstens zwei parallel geschaltete redundante Filterserien, wie im obigen Absatz beschrieben. Dadurch können einzelne Filter stillgelegt werden und der Strom an zu sterilisierenden Produkt auf ein weiteres Kerzenfilter-Gehäuse umgeleitet werden, falls ein Kerzenfilter-Gehäuse seine Standzeit erreicht hat oder die Filterkerze beladen ist. Eine Wartung der Kerzenfilter-Gehäuse ist daher ermöglicht, ohne dass die Produktion von sterilem Medium unterbrochen werden müsste.

Weitere Vorteile und Merkmale ergeben sich aus der nachfolgenden Beschreibung eines Ausführungsbeispiels der Erfindung unter Bezugnahme auf die schematischen Darstellungen. Es zeigen in nicht massstabsgetreuer Darstellung:
- Figur 1:: ein Fliessschema einer Sterilfilterschaltung des Stands der Technik ;
- Figur 2:: einen Querschnitt durch eine Ausführungsform des erfindungsgemässen Kerzenfilter-Gehäuses;
- Figur 3:: eine Frontansicht des Kerzenfilter-Gehäuses;
- Figur 4:: einen Querschnitt durch das Kerzenfilter-Gehäuses in axonometrischer Ansicht;
- Figur 5:: eine axonometrische Ansicht der freigeschnittenen Leitungsführungen, ausgebildet in dem Filterfuss,
- Figur 6:: eine Draufsicht der freigeschnittenen Leitungsführungen, ausgebildet in dem Filterfuss und
- Figur 7:: ein Fliessschema einer Sterilfilterschaltung.

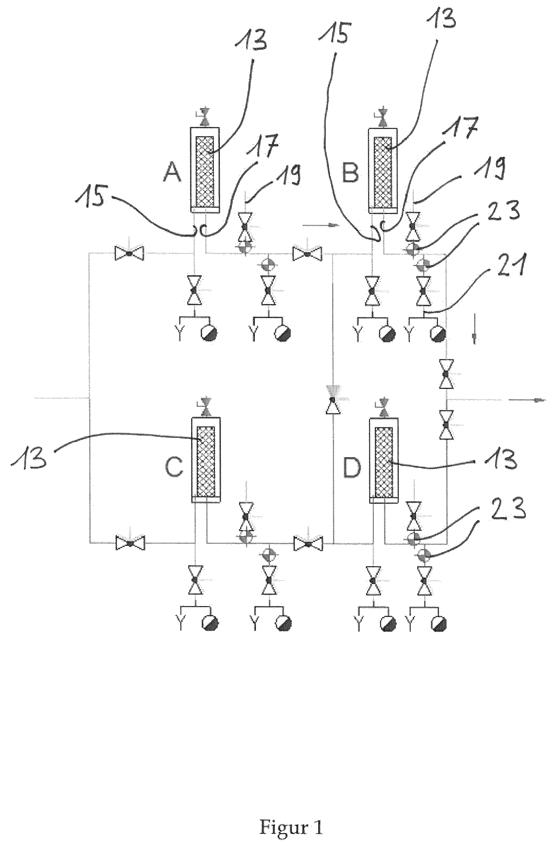

In Figur 1 ist eine Filterschaltung gezeigt, welche aus dem Stand der Technik bekannt ist. Die Kerzenfilter-Gehäuse A und B bzw. C und D sind in Serie geschaltet. Die Filter A und C sind als Vorfilter beispielsweise mit einer Porengrösse von 0,45 µm ausgelegt. Die nachgeschalteten Kerzenfilter-Gehäuse B und D sind als Sterilfilter mit einer Porengrösse von 0,22 µm ausgestattet. Um die Filter A/B bzw. C/D stilllegen zu können, beispielsweise für Wartungszwecke, und die Sterilfiltration trotzdem weiterfahren zu können, sind die Filtergruppen A/B und C/D redundant parallel geschaltet.

Ist eine Dampfsterilisation des Gesamtsystems durchzuführen, sind die maximal möglichen Druckdifferenzen der eingesetzten Filter A, B, C, D zu beachten. Um den für die Filter erträglichen Differenzdruck ohne diese zu zerstören einhalten zu können, ist eine zusätzliche Dampfzufuhr 19 nach der Vorfiltration und nach der Sterilfiltration vorzusehen. Zusätzlich sind Kondensatableitungen 21 vorzusehen. Ein zu hoher Differenzdruck zwischen Aussen- und Innenseite der Filterkerzen 13 kann auch dadurch vermieden werden, dass eine Bypass-Leitung den Zulauf 15 mit dem Ablauf 17 verbindet.

Nachteilig sind bei den Vorkehrungen nach der Sterilfiltration eingebrachte Ventile und Toträume, die systembedingt grösser 3 x D sein müssen. Die Toträume sind in Figur 1 mit dem Bezugszeichen 23 gekennzeichnet. Nachteilig ist auch, dass Ventile, welche nach der Sterilfiltration eingebaut sind, auf Fehlfunktion nicht überwacht werden können. D.h. eine Leckage eines Dichtsitzes, oder Druckspitzen, welche zur Ventilöffnung führen, sind nicht erkennbar und stellen ein Risiko für einen durchgängig sterilen Betrieb dar.

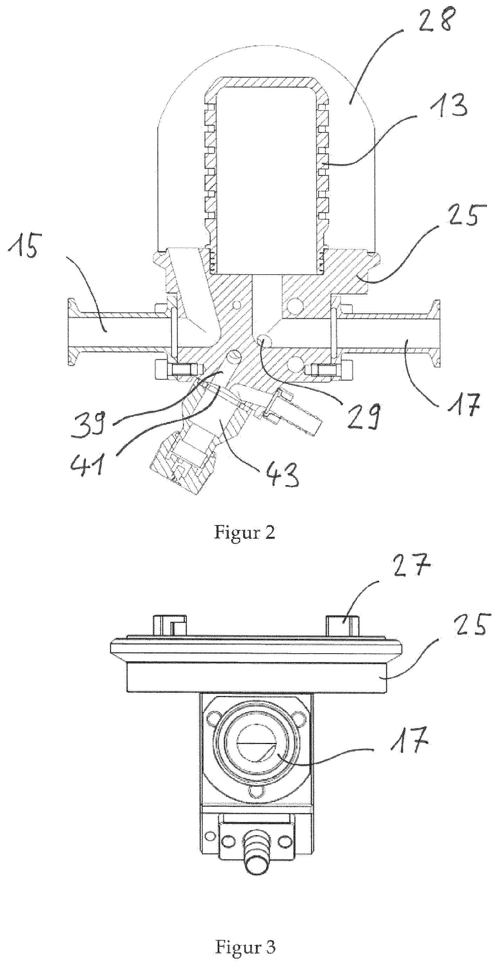

In den Figuren 2 bis 4 ist ein Filterfuss 25 gemäss der Erfindung gezeigt. An dem Filterfuss 25 sind ein Zulauf 15 und ein Ablauf 17 vorgesehen. Eine handelsübliche Filterkerze 13 ist mittels eines Bajonettverschlusses 27 auswechselbar an dem Filterfuss 25 gehalten. Das Medium durchdringt über den Zulauf 15 die Filterkerze 13 von aussen nach innen und verlässt über den Ablauf 17 gereinigt den Filterfuss 25. Zur dichtenden Abdeckung der Filterkerze ist eine Gehäuseglocke 28 vorgesehen, welche abnehmbar an dem Filterfuss 25 befestigbar ist.

Zur Vorort-Reinigung werden CIP (cleaning in place), DIP (drying in place) und SIP (sterilization in place) Verfahren angewendet. Die Reinigungsströme können nicht ausschliesslich über die Filterkerze 13 geführt werden, da der Differenzdruck von 0,2 bar zwischen Aussen- und Innenseite der Filterkerze 13 überschritten werden würde. Eine solche Drucküberschreitung kann zur Zerstörung der Filterkerze 13 führen. Die BypassLeitung 29 ist keine separate Leitung ausserhalb des Filterfusses 25, sondern ist in den Filterfuss 25 vollständig integriert. Die Bypass-Leitung 29 ist durch das Vorsehen von Bohrungen 31 und Fräsungen 33 an dem Filterfuss 25 realisiert. Durch die Ausbildung der Bypass-Leitung 29 in dem Filterfuss 25 können die Toträume besonders kurz gehalten werden und besitzen eine Tiefe von weniger als 3 x D (also dem Durchmesser einer an den Totraum angrenzenden Leitung oder Bohrung).

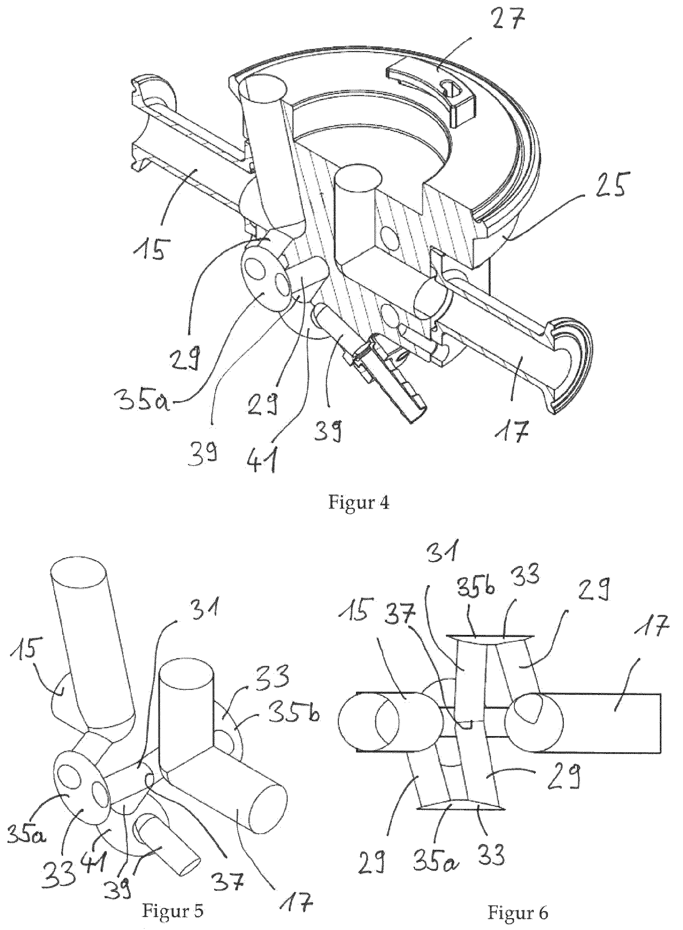

Ein möglicher Verlauf der Bypass-Leitung 29 bzw. der Bohrungen 31 und Fräsungen 33 ist in den Figuren 5 und 6 gezeigt. Zur Durchflussregelung der Bypass-Leitung 29 sind erste Ventilsitze 35a,35b in Gestalt einer Kugelkalotte in den Filterfuss 25 gefräst. Die Ventilsitze 35a,35b können mit ersten Membranventilen zusammenwirken, indem der Durchfluss durch die Bypass-Leitung 29 durch Auf- und Zuschrauben der Membranventile regelbar ist. Sind die Membranventile geschlossen, so erfolgt der gesamte Durchfluss vollständig über die Filterkerze 13.

Die Leckage-Leitung 29 weist im Bereich ihrer Verbindung zwischen den Ventilsitzen 35a,35b eine tiefsten Punkt 37 auf. Der tiefste Punkt 37 ist dadurch gebildet, dass die Bypass-Leitung 29 ausgehend von den Ventilsitzen beidseitig zur Mitte des Filterfusses 25 abfällt.

Am tiefsten Punkt 37 ist die Bypass-Leitung 25 mit einer Leckage-Leitung 39 verbunden. Die Leckage-Leitung 39 umfasst einen zweiten Ventilsitz 41. Der zweite Ventilsitz 41 wirkt mit einem zweiten Membranventil 43 zusammen. Die Leckage-Leitung 39 gibt Auskunft darüber, ob die ersten Membranventile dicht sind oder sich in einer falschen Stellung befinden. Die Dichtheit und Funktion des Kerzenfilters kann daher während der Sterilfiltration jederzeit überprüft werden, indem kontrolliert wird, ob die Leckage-Leitung 39 frei von Medium ist. Dies ist insbesondere bei den hohen Reinheitsanforderungen bei einer Sterilfiltration ein grosser Vorteil. Die Leckage-Leitung 39 kann auch als Kondensatableitung während oder nach einer Dampfreinigung des Kerzenfilters dienen, oder kann zur Restentleerung herangezogen werden.

Der Filterfuss ist bevorzugt aus dem Vollen aus einem einstückigen Edelstahlblock gefertigt. Dabei entstehen die Bypass-Leitung 29, die Leckage-Leitung 39 und die Ventilsitze 35a,35b,41 durch Bohrungen und Fräsungen an dem Metallblock.

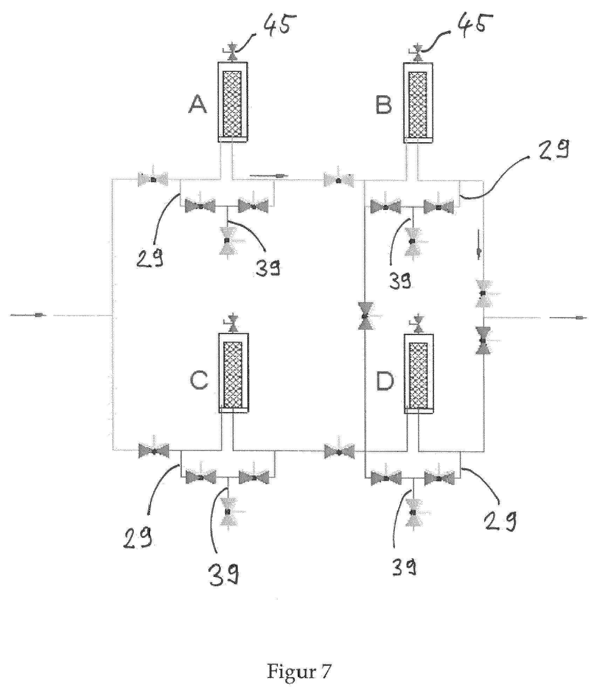

In der Figur 7 ist ein Fliessschema einer Sterilfilterschaltung gezeigt. Wie im ersten Absatz beschrieben, umfasst eine derartige Filterschaltung zwei Paare eines in Serie geschalteten Vorfilters A bzw. C und einem nachgeschalteten Sterilfilter B bzw. D. Die redundante Parallelschaltung der Filterserien A/B und C/D ermöglicht die Wartung beispielsweise der Filterserie A/B, ohne dass die Produktion des sterilen Produktes unterbrochen werden würde, indem der Produktfluss auf die Filterserie C/D umschaltbar ist.

Der Vergleich der Figuren 1 und 7 zeigt, dass durch die in dem Filterfuss 25 ausgebildete Bypass-Leitung 29 sich eine Dampfzufuhr 19 und eine Kondensatableitung 21 erübrigen. Die Toträume 23 sind demzufolge bei dem erfinderischen Kerzenfilter-Gehäuse nicht vorhanden.

Die Filterschaltung gemäss der Figur 7 erlaubt die Sterilfiltration durch die Filterserie A/B oder C/D bei geschlossener Bypass-Leitung 29. Durch das ständig geöffnete zweite Membranventile 43 ist jederzeit überprüfbar, ob die Membranventile der Bypass-Leitung 29 dicht sind und Fehlstellungen und Druckspitzen vorhanden sind. Sollten die ersten Membranventile der Bypass-Leitung 29 undicht sein, so tritt Medium zwangsläufig aus der Leckage-Leitung 39 aus.

Durch Öffnen aller Membranventile, lassen sich die Filterserien CIP, DIP und SIP reinigen. Durch Öffnen eines an der Gehäusglocke 28 vorgesehenen Entlüftungsventils 45, eines der ersten Membranventile und des zweiten Membranventils 43 lassen sich die Filter einfach entleeren. In dieser Ventilstellung lässt sich auch ein Inlineintegritätstest nach der Flüssigsterilisation und vor und nach der Dampfsterilisation durchführen.

Die Integrität des sterilisierten Filters sollte vor und unmittelbar nach der Verwendung durch eine geeignete Methode, z.B. den Gasdiffusionstest (diffusive flowtest, z.B. forward flow Test) ermittelt werden. Ein mit Netzflüssigkeit benetzter Sterilfilter wird mit einem Prüfgas getestet. Wenn Gas durch den benetzten Filter diffundiert wird die entsprechende Gasmenge, die einem bestimmten Volumen entspricht, präzise bestimmt und definiert damit die spezielle Durchflussrate. Diese Durchflussrate ist bei einem integeren Sterilfilter kleiner als die vorgängig durch den Filterhersteller definierte Durchflussrate. Der Test findet bei einem konstanten Prüfdruck und einer konstanten bzw. kontrollierten Temperatur statt. Durch den Aufbau des Kerzenfilter-Gehäuses ist eine Benetzung des Sterilfilters auch direkt nach der Dampfsterilisation mit einem geeigneten Filtertestgerät möglich. Hier muss über die Benetzung bzw. die Spülung des Sterilfilters sichergestellt werden, dass eine Temperatur bei Raumbedingungen (ca. 22°C) konstant gehalten werden. Durch diese Möglichkeit kann ein inline-Filtertest den Sterilfilter in dem Kerzenfilter-Gehäuse in sehr kurzer Zeit sicher überprüfen. Werden wie bei den Filterserien A/B und C/D jeweils der Vor- (A,C) als auch der Sterilfilter (B,D) inline getestet, so ist sicherzustellen, dass auf der sterilen Seite die diffundierte Gasmenge entweichen kann. Hierfür muss für den Filtertest an den Vorfiltern (A,C) an den Sterilfiltern (B,D) das Ventil 45 geöffnet sein. Der Inlinetest der Sterilfilter (B,D) ist wie gehabt, d.h. nach Standardbedingungen beschrieben durch den Filterhersteller.

Die in Figur 7 gezeigte Sterilfilterschaltung ermöglicht es auch durch Vorhandensein der redundanten Filterserie C/D auf diese umzuschalten, wenn die Filterserie A/B ihre Standzeit erreicht hat. Denkbar ist es auch, dass die Sterilfiltration über die Filter A und B oder C und B gefahren wird, wenn die Standzeit der Sterilfilter B oder D erreicht ist.

### Legende:

- A, B, C, D: Kerzenfilter-Gehäuse
- 13: Filterkerze
- 15: Zulauf
- 17: Ablauf
- 19: Dampfzufuhr
- 21: Kondensatableitung
- 23: Toträume
- 25: Filterfuss
- 27: Bajonettverschluss
- 28: Gehäuseglocke
- 29: Bypass-Leitung
- 31: Bohrungen
- 33: Fräsungen
- 35a, 35b: Ventilsitze
- 37: Tiefster Punkt der Bypass-Leitung
- 39: Leckage-Leitung
- 41: Zweiter Ventilsitz
- 43: Zweites Membranventil
- 45: Entlüftungsventil

## Patentansprüche

1. Kerzenfilter-Gehäuse (A, B, C, D) zur Herstellung eines sterilen Mediums,
- in welchem eine Filterkerze (13) einsetzbar ist,
mit
- einem Filterfuss (25) an welchem die Filterkerze (13) austauschbar gehalten werden kann und an welchem ein Zulauf (15) und ein Ablauf (17) für das Medium vorhanden ist,
- einer Gehäuseglocke (28), in welcher die Filterkerze (13) dichtend aufnehmbar ist und welche an dem Filterfuss (25) abnehmbar befestigt ist und
- einer Bypass-Leitung (29), welche den Zulauf (15) und den Ablauf (17) miteinander verbindet,
**dadurch gekennzeichnet,**
**dass** die Bypass-Leitung (29) in dem Filterfuss (25) ausgebildet ist.

2. Kerzenfilter-Gehäuse nach Aspruch 1, **dadurch gekennzeichnet, dass** die Bypass-Leitung (29) durch eine Mehrzahl von an dem Filterfuss (25) vorgesehenen Bohrungen und/oder Fräsungen realisiert ist.

3. Kerzenfilter-Gehäuse nach Anspruch 1 oder 2, **dadurch gekennzeichnet, dass** in die Bypass-Leitung (29) wenigstens ein Öffnungs- bzw. Schliessventil integriert ist, welches derart ausgelegt ist, dass es beim maximal auftretenden Mediendruck im geschlossenen Zustand den Zulauf (15) und den Ablauf (17) gegenüber der Bypass-Leitung (29) verschliesst.

4. Kerzenfilter-Gehäuse nach einem der Ansprüche 1 bis 3, **dadurch gekennzeichnet, dass** an dem Filterfuss (25) wenigstens ein Ventilsitz (35a,35b) ausgeformt ist, welcher mit einem ersten Membranventil derart zusammen wirkt, dass die By-pass-Leitung (29) geschlossen und geöffnet werden kann.

5. Kerzenfilter-Gehäuse nach einem der Ansprüche 1 bis 4, **dadurch gekennzeichnet, dass** der Filterfuss (25) aus dem Vollen gearbeitet ist.

6. Kerzenfilter-Gehäuse nach einem der Ansprüche 1 bis 5, **dadurch gekennzeichnet, dass** der Filterfuss (25) einstückig ist.

7. Kerzenfilter-Gehäuse nach einem der Ansprüche 1 bis 6, **dadurch gekennzeichnet, dass** an dem Filterfuss (25) eine Leckage-Leitung (39) vorgesehen ist, welche mit der Bypass-Leitung (29) in Verbindung steht.

8. Kerzenfilter-Gehäuse nach Anspruch 7, **dadurch gekennzeichnet, dass** die Leckage-Leitung (39) in dem Filterfuss (25) ausgebildet ist.

9. Kerzenfilter-Gehäuse nach einem der Ansprüche 7 oder 8, **dadurch gekennzeichnet, dass** die Leckage-Leitung (39) durch eine Mehrzahl von an dem Filterfuss (25) vorgesehenen Bohrungen und/oder Fräsungen realisiert ist.

10. Kerzenfilter-Gehäuse nach einem der Ansprüche 7 bis 9, **dadurch gekennzeichnet, dass** die Bypass-Leitung (29) einen tiefsten Punkt (37) aufweist, an welchem die Leckage-Leitung (39) mit der Bypass-Leitung (29) verbunden ist.

11. Kerzenfilter-Gehäuse nach einem der Ansprüche 7 bis 10, **dadurch gekennzeichnet, dass** die Leckage-Leitung (29) mit einem zweiten Membranventil (43) geöffnet und geschlossen werden kann.

12. Kerzenfilter-Gehäuse nach einem der Ansprüche 1 bis 11, **dadurch gekennzeichnet, dass** der Filterfuss (25) Toträume aufweist, deren Tiefe kleiner als der dreifache Durchmesser der angrenzenden Bohrungsdurchmesser ist.

13. Kerzenfilter-Gehäuse nach einem der Ansprüche 7 bis 12, **dadurch gekennzeichnet, dass** die Leckage-Leitung (39) als Kondensatableitung dient.

14. Kerzenfilter-Gehäuse nach einem der Ansprüche 1 bis 14, **dadurch gekennzeichnet, dass** das Kerzenfilter-Gehäuse selbstentleerend ist.

15. Filtergruppe mit wenigstens einem ersten und zweiten in Serie geschalteten Kerzenfilter-Gehäuse (A,B,C,D) gemäss einem der vorangehenden Ansprüche, wobei der erste Filter ein Vorfilter (A,C) ist und der zweite Filter ein Sterilfilter (B,D) ist.
